# EUROPÄISCHE PATENTSCHRIFT

(11) **EP 1 372 552 B1**
(45) Veröffentlichungstag und Bekanntmachung des Hinweises auf die Patenterteilung: **01.03.2017**
(21) Anmeldenummer: 02714194.4
(22) Anmeldetag: 26.03.2002
(51) Int. Cl.: A61F 9/007

(54) **VORRICHTUNG ZUR BEARBEITUNG UND DIAGNOSE VON AUGENGEWEBE**
DEVICE FOR THE TREATMENT OF TISSUES OF THE EYE AND FOR DIAGNOSIS THEREOF
DISPOSITIF POUR TRAITEMENT DE TISSUS OCULAIRES ET DE DIAGNOSTIC

(30) Priorität: 27.03.2001 DE 10115751; 28.09.2001 DE 10148783
(43) Veröffentlichungstag der Anmeldung: 02.01.2004
(73) Patentinhaber: WaveLight GmbH, 91058 Erlangen (DE)
(72) Erfinder: KOENIG, Karsten, 07778 Neuengönna (DE)
(74) Vertreter: von Hellfeld, Axel
(86) Internationale Anmeldenummer: PCT/EP2002/003370
(87) Internationale Veröffentlichungsnummer: WO 2002/076355

(56) Entgegenhaltungen:
- EP-A- 0 850 614
- EP-A- 0 903 133
- WO-A-01/19303
- WO-A-98/55035
- DE-A- 19 837 932
- US-A- 5 098 426
- US-A- 5 984 916
- US-A- 6 099 521
- US-A- 6 146 375
- JUHASZ T ET AL: "TIME-RESOLVED OBSERVATIONS OF SHOCK WAVES AND CAVITATION BUBBLES GENERATED BY FEMTOSECOND LASER PULSES IN CORNEAL TISSUE AND WATER" LASERS IN SURGERY AND MEDICINE, WILEY- LISS, NEW YORK, US, Bd. 19, Nr. 1, 1996, Seiten 23-31, XP000597102 ISSN: 0196-8092 in der Anmeldung erwähnt
- LUBATSCHOWSKI H ET AL: "APPLICATION OF ULTRASHORT LASER PULSES FOR INTRASTROMAL REFRACTIVE SURGERY", GRAEFE'S ARCHIVE FOR CLINICAL AND EXPERIMENTAL OPHTHALMOLOGY, SPRINGER VERLAG, XX, vol. 238, 1 January 2000 (2000-01-01), pages 33-39, XP001014554, ISSN: 0721-832X, DOI: 10.1007/S004170050006

## Beschreibung

Die Erfindung betrifft eine Vorrichtung zur minimal- bis nicht-invasiven Augenchirurgie durch optische Bearbeitung des Gewebes mittels Laserstrahlung. Vorzugsweise dient die Vorrichtung der refraktiven Corneachirurgie zur Behandlung von Fehlsichtigkeit, wobei auch eine "on-line"-Diagnose und -Therapiekontrolle erfolgen kann. Die Anordnung kann auch für andere Eingriffe im Auge genutzt werden, z.B. für die Glaukomtherapie, um durch laserinduzierte Gewebedurchtrennung (Kanalbohrung) den geregelten Abfluß des Kammerwassers wieder zu ermöglichen oder die Kammerwasserproduktion durch partielle Entfernung des Ziliarkörpers zu reduzieren. Zudem können Zysten und Tumore und sonstige pathologische Gewebeveränderungen am und im Auge diagnostiziert und laserbehandelt (punktiert) werden.

Die refraktive Corneachirurgie erfolgt bislang üblicherweise mit invasiven mechanischen Methoden, mittels Laserstrahlung oder mit einer Kombination von mechanischen Methoden mit einer Laserbehandlung.

Bei der Behandlung mit Laserstrahlung ohne mechanische Methoden wird typischerweise ein Excimerlaser mit einer gut absorbierenden Laserwellenlänge im ultravioletten (UV) Bereich mit Impulslängen im Nanosekundenbereich eingesetzt. Der Abtragungsprozeß basiert auf der sogenannten Photoablation. Bei der Behandlung mit dem Excimerlaser wird von der Oberfläche des Cornea, beginnend an der sogenannten Epithelschicht, ca. 100 gm tief Gewebe abgetragen, um eine Brechkraftkorrektur zu erreichen. Nachteil ist die relativ schlechte Verheilung durch optische Entfernung der Epithelschicht.

Dagegen wird beim sogenannten LASIK-Verfahren zunächst mit einer mechanischen Vorrichtung (Mikrokeratom) ein oberer Teil der Cornea teilweise "abgehobelt". Die teilweise abgetrennte Homhautschicht, der sogenannte Flap, wird zur Seite geklappt und gibt die darunter liegende Gewebeschicht zum Entfernen von Gewebe frei. Es folgt die optische Gewebeabtragung mittels UV-Excimerlaser. Nach der Laserbehandlung wird der Flap zurückgeklappt und haftet an der Cornea durch Adhäsisionskräfte. Die so erzeugte Abflachung der Cornea dient der Korrektur der Kurzsichtigkeit. Nachteil dieser Behandlung ist der relativ hohe Anteil (typischerweise 5%) von Komplikationen durch den anfänglichen mechanischen Eingriff. Zudem kann durch mechanische Einwirkung, z.B. heftiges Reiben, der Flap auch nach langer Zeit nach der Therapie wieder verrutschen.

Von besonderem Interesse ist daher der Versuch einer minimal- bis nicht-invasiven optischen Therapie im Inneren der Cornea, insbesondere in der sogenannten Stromaschicht, ohne Verletzung der Augenoberfläche. Dies kann prinzipiell durch fokussierte Laserstrahlung hoher Intensität mit Wellenlängen im sichtbaren und nahen infraroten (NIR) WellenlängenBereich bis etwa 1200 nm erfolgen.

Der Materialabtrag erfolgt üblicherweise bei extrem hohen Intensitäten im Größenordnungsbereich von GW/cm und TW/cm durch Ionisation von Biomolekülen infolge nichtresonanter Multiphotonen-Absorption. Die ersten so erzeugten freien Elektronen lösen einen Prozeß aus, der über lawinenartige Amplifikationseffekte infolge der Wechselwirkung mit dem elektromagnetischen Feld der Laserstrahlung und damit verbundener Energieabsorption durch inverse Bremsstrahlung, zum laserinduzierten optischen Durchbruch und Plasmabildung fuhrt. Durch die rapide Ausdehnung des Plasmas entsteht ein dynamisches Hochdruckgebiet, das die Entstehung einer radial sich ausdehnenden Schockwelle bewirkt. Der durch Schockwellen und Formation von Blasen (Kavitätsblasen, Gasblasen) bewirkte Anteil am Materialabtrag wird als Photodisruption bezeichnet [Juhasz et al. IEEE Journal of Selected Topics in Quantum Electronics 5 (1999) 902-909]. Optomechanisch können Ablationsfragmente auch aus dem Interaktionsgebiet transportiert werden [Loesel et al. Appl. Phys. B 66 (1998) 121-128],

Bislang erfolgten Versuche mit Nanosekundenimpulsen, Pikosekundenimpulsen und Femtosekundenimpulsen [z.B. Krasnov. Arch. Ophthalmol. 92(1974)37-41; Stern et al. Arch. Ophthalmol. 107 (1989) 587-592; Niemz et al. Lasers Light Ophthalmol. 5 (1993) 149155; Vogel et al. Invest. Light Ophthalmol. 5 (1993) 149-155; Juhasz et al. Lasers Surg. Med. 19 (1996) 23-29].

Nanosekundenimpulse erfordern hohe Impulsenergien und zeigen infolge eines hohen Anteils an hoher mechanischer Energie nur begrenzte therapeutische Möglichkeiten im Bereich der Corneachirurgie (Steinert and Puliafito. The Nd:YAG laser in opthalmology. Philadelphia, PA: W.B. Saunders, 1985: 11-21). Bei der Verwendung von kürzeren Impulsen sinkt die Schwelle für den therapeutischen Durchbruch. Durch die Verwendung von Impulsen geringer Energie kann der Anteil destruktiver mechanischer Energie reduziert werden, wie durch die Verwendung von Pikosekundenimpulsen demonstriert wurde. Jedoch wurde auch hier keine optimale Behandlung erreicht, was insbesondere auf die Ausbildung von Blasen zurückzufuhren ist [Niemz et al. Lasers Light Ophthalmol 5 (1993) 149-155; Gimpel et al. Int. Ophthalmol. Clin. 37(1997)95-102; Ito et al. J. Refract. Surg. 12(1996)721-728]. So beträgt der Durchmesser von Kavitationsblasen bei Verwendung von Nanosekundenimpulsen typischerweise 1 mm bis 2 mm, bei Pikosekundenimpulsen 0,2 mm bis 0,5 mm, Vogel et al. Proc. SPIE 1877 (1993) 312-322]. Günstigere therapeutische Wirkungen erhofft man sich durch die Verwendung von Femtosekundenimpulsen.

Bisherige Untersuchungen zur refraktiven Corneachirurgie mit Femtosekundenimpulsen basieren auf der Verwendung von Impulsen mit Impulsenergien im Mikrojoule- und Millijoule-Bereich bei Impulsfolgefrequenzen im Hz- bis kHz-Bereich und Laserbeleuchtungsspots mit einem Durchmesser von mehreren Mikrometern [z.B. Kurtz et al. J. Refract. Surg. 13 (1997) 653-658], So beschreibt Kurtz et al. eine Anordnung, die durch eine Folgefrequenz von 10 Hz, einem Beleuchtungsspot von 26 µm Durchmesser, Impulsenergien bis 10 mJ und variierbarer Impulsdauer gekennzeichnet ist [Kurtz et al., J. Refract. Surg. 13 (1997) 653-658].

Lubatschowski et al. nutzten ein Lasersystem mit einer Folgefrequenz von 1000 Hz, einer maximalen

Impulsenergie von 1 mJ und einem Beleuchtungsspotdurchmesser von 7 µm [Graefe's Arch. Clin. Exp. Ophthalmol. 238 (2000) 33-39], Derartige Anordnungen, die typischerweise aus einem Laseroszillator und einem Verstärker bestehen sowie "Puls-Stretch"-Module und Puls-Kompressions"-Module enthalten, sind platz-, betreuungs-und kostenintensiv. Dieser Stand der Technik ist ausschließlich auf den therapeutischen Aspekt ausgerichtet. Messungen am Augengewebe sind nicht vorgesehen. Dort wird ein Laserpulsdurchmesser im Bereich von 5-10 µm vorgeschlagen. Die Folgefrequenz (Repetitionsrate) liegt bei 1 kHz. Die Pulsenergien liegen deutlich über dem Nanojoulebereich. Mit diesen Anordnungen und Laserparametern können Schnitte im Inneren der Cornea mit einer Breite von typischerweise mehr als 10 µm erzeugt und so Material abgetragen werden. Zudem kann ein Flap optisch erzeugt werden. Ein entsprechendes Gerät befindet sich auf dem Markt. Dabei werden Femtosekundenimpulse der Wellenlänge von 1053 nm genutzt. Die Strahlung wird dabei auf einen Spot mit dem Durchmesser von 3 µm in das Auge fokussiert und mittels einer Scanningvorrichtung intraokular positioniert. Die Bestrahlungspunkte liegen in einem räumlichen Abstand von mehr als 5 µm dicht nebeneinander in Form einer Spirale, sind jedoch zeitlich versetzt. Material wird vom Inneren bis zur Cornea-Oberfläche derart entfernt, daß unter Zuhilfenahme eines Unterdruckes der mittels Laserstrahlung erzeugte Flap zur Seite geklappt werden kann. Die mechanische Flap-Herstellung entfällt dadurch.

In den Patenten US 5.993.438 und EP 0 903 133 wird ein Verfahren zur intrastromalen photorefraktiven Keratectomy beschrieben, das die Photodisruption von Material im Stroma beschreibt, wobei das durch Photodisruption betroffene Material etwa dem Fokusvolumen mit einem Durchmesser von typischerweise 10 µm bis 25 µm entspricht und die Beleuchtungsspots derart plaziert werden, daß ihr räumlicher Abstand ein bis zwei Durchmessern der erzeugten Blasen entspricht und sie auf die optische Achse bezogen zentralsymmetrische laserbearbeitete Schichten erzeugen, die eine gewünschte Kavität im Stroma erzeugen können. In vorliegender Erfindung wird eine Methode unter Verwendung der Impulsfolgefrequenz im Bereich 10 Hz und 100 kHz beschrieben. Bevorzugte Frequenzen sind 1 kHz bis 10 kHz und ein Beleuchtungsspot mit einen Durchmesser von etwa 10 µm. Die bekannten technischen Lösungen beruhen auf der Anwendung der Photodisruption, also der mechanischen Wirkung von Schockwellen und Blasen. Das photodisruptierte Gewebe soll von der Cornea absorbiert oder aus der Cornea abtransportiert werden.

Im Patent US 6.146.375 wird über die Photodisruption von Gewebe zur Behandlung des Glaukoms mit Femto- und Pikosekundenimpulsen teilweise unter Zuhilfenahme von das Streuverhalten des Auges verändernden chemischen Substanzen berichtet.

Als Nachteil der bisherigen Verfahren mittels Femtosekundenimpulsen erweisen sich die bislang verwendeten relativ hohen Impulsenergien in der Größenordnung von Mikrojoule, die zu unerwünschten mechanischen Wirkungen, insbesondere durch die Wirkung von Blasen, den sogenannten Bubbles, und der damit verbundenen Schockwellen durch den Prozeß der Photodisruption führen. So wird über die Bildung von 25 µm großen Blasen bei Verwendung von 2 µJ-Impulsen mit einer Impulsdauer von 300 fs in Wasser und über Koagulationen von Kollagen innerhalb der Interaktionszone berichtet [Lubatschowski et al. Graefe's Arch. Clin. Exp. Ophthalmol. 238 (2000) 33-39]. Zudem können bei diesen relativ hohen Impulsenergien Selbstfokussierungseffekte induziert werden, die zu unerwünschten Schäden im umgebenen Gewebe führen können. Auch erfordert die Verwendung dieser relativ hohen Pulsenergien aufwendige kosten- und bedienungsintensive Lasersysteme mit Verstärkern.

Von Nachteil ist auch, daß bisherige Femtosekunden-Lasersysteme zur Corneachirurgie keine Analyse hoher Auflösung der Laserbehandlung ermöglichen. Üblicherweise werden gesonderte optische Systeme für eine Diagnostik genutzt (z.B. Arashima et al., EP 0 850 614 A1). In dieser Schrift wird ein System beschrieben, das einen Laser für die Corneablation, ein zusätzliches Beleuchtungssystem und eine Photographiereinrichtung umfaßt.

In der Patentschrift US 5,984,916 wird ein Verfahren und eine Anordnung zur Laser-Augenchirurgie beschrieben, welche auf der Anwendung von Bestrahlungsspots von ca. 10 µm, Pulsfrequenzen bis zu 100 KHz und Energiedichten von 0,2 - 5 µJ/10 µm² basieren. Derartige Energiedichten und Pulsfrequenzen setzen jedoch die Verwendung von aufwendigen Lasersystemen mit Amplifier, Pulsstretching und PulskompressionsEinheiten sowie Pulsenergien im Bereich grösser 0,2 µJ voraus. Ein integriertes Diagnosesystem ist nicht vorgesehen.

Gegenüber dem Stand der Technik gemäß der oben zitierten Abend von Lubatschowski et al. liegt der vorliegenden Erfindung die Aufgabe zugrunde, bei einer Vorrichtung zu minimal- bis nicht nicht-invasiven optischen Bearbeitung von Augengewebe die Vielseitigkeit des Systems zu erweitern.

Die erfindungsgemäße Vorrichtung ist im Patentanspruch 1 beschrieben.

Die abhängigen Ansprüche beschreiben vorteilhafte Weiterbildungen der Vorrichtung.

Die Vorrichtung ermöglichteine bislang unerreichte hohe Präzision mit möglichen Schnittbreiten im Bereich kleiner 2 µm auszeichnet, ohne daß eine durch Photodisruption und Selbstfokussierung erzeugte signifikante mechanische Beeinträchtigung des umliegenden Gewebes auftritt. Die Verwendung kostengünstiger und leicht zu bedienender Systeme soll möglich sein. Zudem soll die gleiche Anordnung eine dreidimensionale Bilderstellung (Imaging) des Gewebes zur Diagnose, zur Targetanalyse, zur optischen on-line Behandlungskontrolle und zur dreidimensionalen hochauflösenden optischen Analyse der Laserbehandlung ermöglichen.

Die Wirksamkeit der Erfindung wird nachfolgend an Ausführungsbeispielen nachgewiesen und ihre Funktion näher erläutert. Es zeigen:
- Fig. 1A:: HE-gefärbte Gefrierschnitte eines Gebietes mit Laserschnitten, welche das präzise Schneiden im Stroma eines Schweineauges mit Sub-Nanojoule Femtosekunden-Laserimpulsen belegen. Eine Messung ergab typische Schnittbreiten im Bereich von 0.3 µm bis 1 µm.
- Fig. 1B:: Remissionsaufnahmen unmittelbar nach 5 erfolgten Schnitten im Stroma eines Schweineauges mit jeweils 20 ms Gesamt-Verweilzeit des Strahls per Pixel und einer 512 Pixel-Linien-Abtastung.
- Fig. 2:: Aufnahmen der mit einer mittleren Wellenlänge von 800 nm angeregten Autofluoreszenz und Second Harmonie Generation (SHG) mit hoher räumlicher Auflösung in verschiedenen Gewebetiefen, d.h. in z- Richtung, eines Schweineauges. Deutlich sind die verschiedenen Gewebeschichten der Cornea und einzelne Zellen erkennbar.
- Fig. 3:: Fluoreszenzaufnahme 2 s nach erfolgter Lasertherapie mit 2 ms Gesamt-Verweilzeit des Strahls pro Pixel. Das lumineszierende Gebiet entlang des Schnittes weist eine Breite von ca. 0.8 µm auf. Das einzelne größere leuchtende Areal stellt die Lumineszenz einer Blase dar.
- Fig. 4:: Remissionsaufnahmen, welche 4 s, 15 s, 30 s und 45 s nach Materialabtragung mit einem "Linescan 6" erfolgten und Informationen über die Bubble-Kinetik ergeben. Demnach liegt die Lebensdauer dieser "Bubbles" im Bereich von weniger als einer halben Minute.
- Fig. 5:: Ein prinzipielle Darstellung einer erfindungsgemäßen Anordnung mit einem einfachen Laserstrahl.
- Fig. 6:: Darstellung wie Fig. 5, jedoch mit einem in mehrere Einzelstrahlen aufgeteilten Laserstrahl.

Es wird zur minimal- bis nicht-invasiven optischen Bearbeitung, zur dreidimensionalen Bilderstellung, zur optischen on-line Behandlungskontrolle und zur dreidimensionalen hochauflösenden optischen Analyse der Laserbehandlung von Geweben des Auges, insbesondere der Cornea, fokussierte Strahlung im Spektralbereich von 500 nm bis 1200 nm, bestehend aus Femtosekunden-Impulsen mit einer Impulsenergie im Pikojoule-Bereich und Nanojoule-Bereich mit hoher Folgefrequenz im MHz-Bereich und Bestrahlungsspots mit einem Durchmesser kleiner 5 µm, vorzugsweise kleiner 1 µm, die über das zu bearbeitende Target mit einem typischen Abstand kleiner 5 µm verschoben werden, verwendet, wodurch eine präzise Bearbeitung durch selektive unmittelbare Zerstörung von einzelnen Zellen oder Zellbestandteilen oder einzelnen intraokularen Gewebestrukturen ohne irreversible Zerstörung umliegender Gewebeareale ermöglicht, die dreidimensionale Aufnahme des zu behandelnden oder behandelten Gewebes oder einzelner Zellen oder einzelner Zellbestandteile vor und nach der Lasertherapie durch Detektion der Fluoreszenz, bevorzugt der nichtlinear angeregten Autofluoreszenz, oder der Remission ermöglicht sowie eine on-line Therapiekontrolle durch räumlich und/oder zeitlich aufgelöste on-line Detektion des Plasmaleuchtens ermöglicht werden.

Es kann die Lasertherapie und die dreidimensionale Bilderstellung (Imaging) des Gewebes zur Targetanalyse, zur optischen on-line-Behandlungskontrolle und zur dreidimensionalen hochauflösenden optischen Analyse der Laserbehandlung mit nur einer einzigen Anordnung realisiert werden. Erfindungsgemäß kommt eine Anordnung zur Bearbeitung und zur Diagnostik zum Einsatz, die aus einem kompakten Femtosekundenlaser ohne Verstärker im Bereich 500 nm bis 1200 nm, einem Strahlführungssystem einschließlich Scaneinrichtung, einem Strahlaufweiter, einem schnellen Leistungsregler zum Umschalten zwischen Diagnostik (Targetsuche und Wirkungskontrolle) mit Strahlung geringer Leistung und Therapie mit Strahlung hoher Leistung, einem oder mehreren Photonendetektoren, Monitoren, Strahlunterbrecher, sowie geeigneter Steuerung und Hard- und Software besteht. Um eine zeitaufgelöste Detektion der Signale, hervorgerufen durch Remission, Fluoreszenz und Plasmaleuchten mit einer Auflösung im Pikosekundenbereich zu ermöglichen, wird erfindungsgemäß ein schneller Detektor, typischerweise ein schneller PMT, mit einem Modul zur zeitkorrelierten Einzelphotonenzählung gekoppelt. Für eine on-line

Beobachtung von Effekten kann zusätzlich eine Videokamera eingesetzt werden.

Für die Fokussierung der Strahlung Objektive einer numerischen Apertur grösser 0,8, typischerweise grösser 1,0, verwendet und Bestrahlungsspots in einem Abstand kleiner 5 µm, typischerweise kleiner 1 µm, positioniert.

Für die Durchführung der Lasertherapie werden Strahlungsintensitäten T von mehr als 100 GW/cm² verwendet, für die Diagnostik geringere Intensitäten. Die unterschiedlich benötigten Intensitäten werden durch Variation der Laserleistung an der Probe realisiert. Der Leistungsregler muß die Wahl zwischen Diagnostik und Therapie und die Einstellung der jeweils benötigten Lichtintensität in Abhängigkeit von der Tiefe des zu untersuchenden bzw. zu bearbeitenden Gewebeareals ermöglichen.

Es wurde gefunden, daß durch geeignete Femtosekunden-Laserimpulse im Subnanojoule- und Nanojoule-Bereich intraokluare Materialabtragungen erzielt werden können. Dies wurde durch die Verwendung von kompakten, einfach zu bedienenden Lasersystemen möglich. Die Verwendung von aufwendigen Lasersystemen mit Verstärker ist nicht erforderlich. Es konnte eine bislang unerreichbare Präzision von < 1 µm Schnittbreite im Stroma und Epithelgewebe erzielt werden. Dabei konnten einzelne Zellen abgetragen, einzelne Kollagenfasern getrennt oder ganze Gewebebereiche entfernt werden ohne die umliegenden Gewebebereiche durch Photodisruption zu schädigen.

Insbesondere zeigte es sich, daß 170-Femtosekunden-Impulse der Zentralwellenlänge 800 nm, der Folgefrequenz von 80 MHz bei Verwendung einer fokussierenden Optik der numerischen Apertur 1,3, welche Bestrahlungsspots kleiner als 1 µm ermöglicht, bei einer mittleren Leistung von 60 mW, was einer Impulsenergie im Sub-nJ-Bereich entspricht, es ermöglichen, Material in der Cornea abzutragen. Der Bestrahlungsspot wurde mit einem Galvanometer-Scanner auf dem Target verschoben. Die Verschiebung erfolgte in Schritten von weniger als 1 µm, typischerweise weniger als 0,5 µm. Der zeitliche Abstand einer Verschiebung war kleiner als 100 µs. Die Strahlverweilzeit pro

Bestrahlunsspot liegt ebenfalls im Mikrosekundenbereich, typischerweise im Bereich kleiner als 10 ps. Jeder Spot wurde bis zu 5000mal bestrahlt, typischerweise etwa 200 bis 500mal. Es konnten Schnittbreiten kleiner 1 pm erzielt werden, ohne umliegende Zellen des Gewebes zu schädigen. Diese Schnittbreiten konnten sowohl in der Epidermis, in der Bowman- Membran als auch im Stroma erreicht werden.

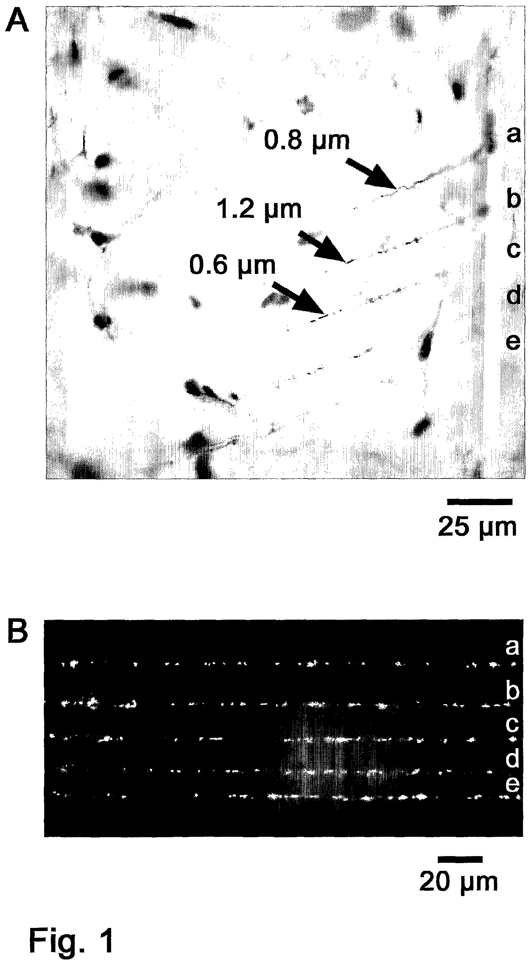

Die Fig. 1A zeigt histologische Uli-gefärbte Gefrier-Gewebeschnitte eines Schweineauges, welche Laser-induzierte Materialentfernungen demonstrieren. Verwendet wurde eine mittlere Leistung von 80 mW. Der Strahl wurde 5mal entlang einer Linie (Linescan) geführt, die Strahlverweilzeit pro Pixel betrug insgesamt 20 ms. Die erzielte Schnittbreite variiert demnach von 0,3 µm bis etwa 1 µm. Es sind keine Anzeichen von thermischer oder mechanischer Schädigung der benachbarten Gewebeareale erkennbar.

Die Fig. 1B demonstriert Remissionsaufnahmen, die mit der gleichen Anordnung unmittelbar nach Durchführung der Materialentfernungen durchgeführt wurden. Es wurde anhand dieser Aufnahmen gefunden, daß durch die Laser-induzierten Materialentfernungen hoch-reflektive Zonen entlang der Schnittkanten entstanden. Diese lassen sich mittels der Laserstrahlung gleicher Wellenlänge, jedoch wesentlich geringer mittlerer Leistung von kleiner 1 mW, unter Verwendung von geeigneten Photonendetektoren dreidimensional abbilden. Die Breite dieser reflektierenden Zonen entlang des Schnittes weist ebenfalls Werte kleiner 1 pm auf und korreliert daher näherungsweise mit der tatsächlichen im histologischen Bild erkennbaren Schnittbreite. Interessanterweise zeigten auch die während des Materialabtrags erzeugten Bubbles eine deutlich sich von der Umgebung unterscheidende meßbare Reflexion. Weniger stark reflektierend, jedoch dennoch gut sichtbar, zeigen die 3D-Remissionsbilder deutlich reflektierende Strukturen einzelner Zellen in der Epithelschicht, insbesondere den stark reflektierenden Zellkern und die Zellmembranen, sowie vermutlich Kollagenstrukturen innerhalb des Stromas.

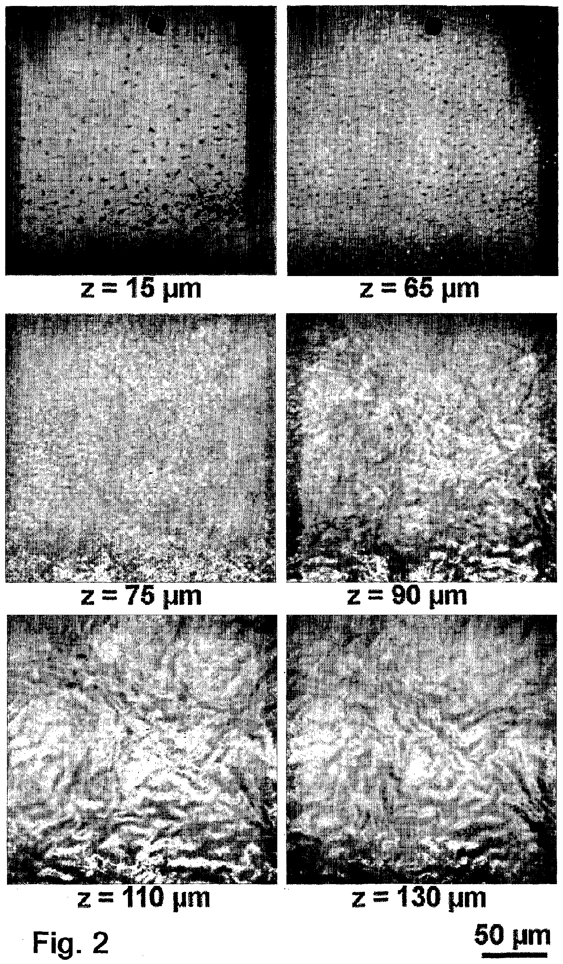

Mit der gleichen Apparatur konnten auch Fluoreszenzaufnahmen erstellt werden. Bei einer mittleren Leistung von 2 mW bis 5 mW konnte durch Multi-Photonen-Anregung von endogenen Fluorophoren im Sub-Femto-liter-Fokusvolumen und Fluoreszenzdetektion mit einem Photomultiplier durch Abscannen von Ebenen in verschiedenen Gewebetiefen ein dreidimensionales Bild der Cornea vor und nach der Laserchirurgie erstellt werden. Insbesondere konnten deutlich die verschiedenen Gewebeschichten der Cornea, nämlich die Epithelschicht, die Bowman-Membran und die Sklera anhand der Autofluoreszenz lokalisiert werden. Die Fig. 2 zeigt entsprechende 800 nm angeregte Autofluoreszenzaufnahmen hoher räumlicher Auflösung in verschiedenen Gewebetiefen eines Schweineauges.

Insbesondere kann durch eine Zweiphotonen-Anregung die Fluoreszenz des reduzierten Koenzyms NAD(P)H sowie von Flavinen dargestellt werden. Anhand der Fluoreszenz können deutlich die einzelnen Zellen lokalisiert werden. Zudem zeigen die Kollagenfasem des Stromas eine deutliche Autofluoreszenz und SHG-Strahlung.

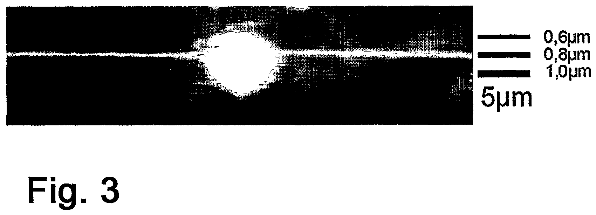

Es wurde auch hier gefunden, daß durch die Laserbehandlung entstehende Bubbles durch Einwirkung von Laserlicht geringer Leistung zu einer Lumineszenz angeregt werden können, die deutlich über der Intensität der Autofluoreszenz liegt. Zudem zeigen die bearbeiteten Areale entlang der Schneidzone eine Autofluoreszenz, die sich von umgebenen Bereichen unterscheidet. Dadurch kann der Behandlungseffekt mit hohem Kontrast deutlich gemacht werden (Fig. 3).

Es konnte das während der Laserbestrahlung erzeugte Plasmaleuchten mit dem gleichen Photomultiplier direkt während der Laserbearbeitung entlang des Bearbeitungsareals detektiert werden. So ist eine Aussage über die Wirkung der intensiven Laserstrahlung ortsaufgelöst möglich und damit eine on-line Therapiekontrolle gegeben.

Wird während der Laserbehandlung eine Weitfeldbeleuchtung des Targets mit Weißlicht oder vorzugsweise mit Licht im nahen Infrarot von einer Halogenlampe bzw. von LEDs genutzt, so können die Effekte der Laserbehandlung, insbesondere die Formation und das Verschwinden von Bubbles, on-line durch Remissionsmessung beispielsweise mit einer 50 Hz-CCD-Kamera detektiert und beispielsweise auf einem Videorekorder oder auf einen PC gespeichert und wiedergegeben werden.

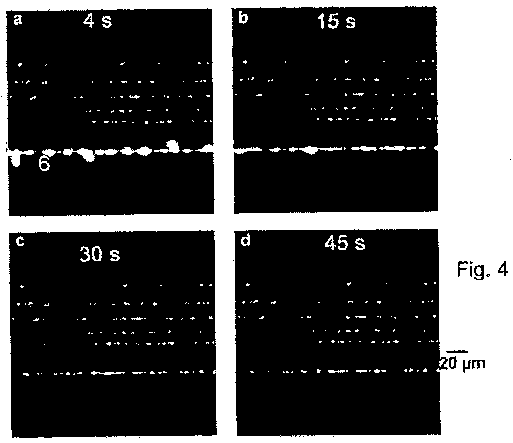

Durch Messung der reflektierten und gestreuten Photonen sowie der Fluoreszenzphotonen unmittelbar nach Durchführung der Lasertherapie können Aussagen zur erzielten Wirkung und zur Schnittbreite getroffen werden. Zudem kann das Auftreten von Bubbles und deren dynamisches Verhalten untersucht werden, wie es die Fig. 4 verdeutlicht. Typischerweise weisen die entstehenden Blasen Abmessungen von weniger als 5 µm auf und verschwinden innerhalb von wenigen Sekunden, wie an den Reflexionsbildern 4 s, 15 s, 30 s und 45 s nach erfolgtem zellenförmigen Materialabtrag (6) dargestellt.

Da bei geeigneten Pulsenergien im Sub-Nanojoule-Bereich nahe den Schwellwerten für den optischen Durchbruch Materialabtragungen durchgeführt werden können und keine Anzeichen von mechanischen Schäden der Umgebung gefunden werden konnten, ist die Materialabtragung möglicherweise nicht auf eine Photodisruption, sondern lediglich auf eine Materialverdampfung durch rein thermische Effekte oder einen fotochemischen Materialabtrag (Aufbrechung von Bindungen durch Multiphotonenabsorption induzierten Energieeintrag) zurückzuführen. Diese Annahme wird durch Untersuchungen unterstützt, die nahe dem Schwellwert Bubbles hervorriefen, die nicht die typischen, durch Photodisruption entstehenden kurzlebigen Kavitätsblasen darstellen [Lubatschowski et al. Graefe's Arch. Clin. Exp. Ophthalmol. 238 (2000) 33-39], ß, ,,

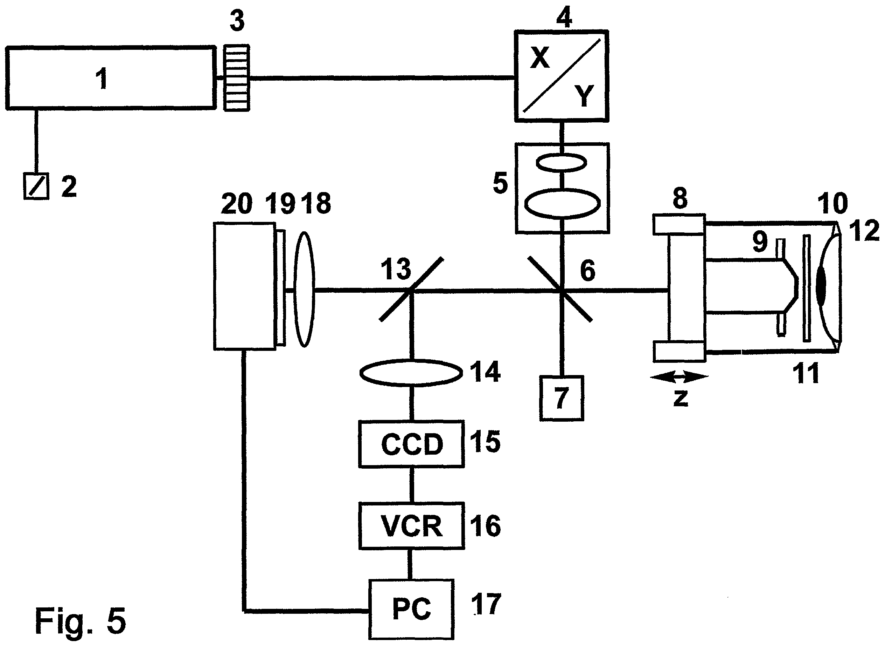

Die Fig. 5 demonstriert die Vorrichtung. Als Bestrahlungsquelle für die Materialabtragung, die Anregung der Fluoreszenz und der Lumineszenz der Bubbles sowie der Gewinnung von Remissionsstrahlung wird ein kompakter Femtosekundenlaser 1 mit hoher Folgefrequenz mit typischen Werten um 80 MHz eingesetzt. Die zentrale Laserwellenlänge liegt im Bereich von 700 nm bis 1200 nm, ein typischer Wert ist 800 nm. Der Betrieb des Lasers 1 ist an einen Fußschalter 2 gekoppelt. Der Laserstrahl trifft auf einen schnellen Schalter 3 mit integriertem Leistungsregler. Dieser Schalter ist typischerweise ein elektrooptischer Schalter mit Schaltzeiten im Mikrosekundenbereich. Er ist zudem in der Lage, die Laserleistung zu variieren und die Ausgangsleistung des Lasers 1 um Größenordnungen zu reduzieren. Der Strahl trifft auf einen Scanner 4, der typischerweise aus zwei Galvanometer-Spiegeln für die x-y-Ablenkung besteht. Der Strahl transmittiert eine Scanner- und Aufweitungsoptik 5, bevor er über einen als Strahlenteiler wirkenden Umlenkspiegel 6 auf die Fokussierungsoptik 9 gelenkt wird. Typischerweise reflektiert der Umlenkspiegel 6 ca. 99% der Strahlung. Die transmittierten Anteile der Strahlung von 1% treffen auf einen Detektor 7, welcher der Leistungsmessung vornimmt und gegebenenfalls ein Triggersignal zur Verfügung stellt. Die Fokussierungsoptik 9 kann mittels eines piezogetriebenen Verstellers 8 mit Nanometergenauigkeit verstellt und so die Fokusebene variiert werden. Eine mechanische Halterung 11 dient der Fixierung der Augenposition und kann ein 170 jim dickes Glasfenster 10 aufnehmen. Der Strahl wird auf das Auge 12 fokussiert. Remittierte bzw. im Auge 12 entstandene Strahlung wird durch den ersten Strahlenteiler 6 zu einem geringen Prozentanteil, typischerweise 1%, transmittiert und durch einen Teilerspiegel 13 als zweitem Strahlenteiler zum einen durch eine Abbildungsoptik 14 auf einen Strahlungsdetektor 15, typischerweise eine CCD-Kamera, geleitet. Das entstehende Bild kann mittels eines Videorekorders 16 und eines Personalcomputers 17 on-line räumlich aufgelöst aufgenommen werden. Lumineszenzstrahlung wird durch die Strahlenteiler 6 und 13, die eine Optik 18 und einen Filter 19 auf einen Strahlungsdetektor 20 geleitet. Dieser Strahlungsdetektor 20 detektiert die Fluoreszenz, die Plasmalumineszenz und die Lumineszenz der Bubbles. Erfindungsgemäß kann dieser Strahlungsdetektor 20 ein Photomultiplier (PMT) mit üblicher Responsezeit, ein schneller PMT in Verbindung mit einem "Single Photon Counting" (SPC)-Modul mit Zeitauflösung im Pikosekundenbereich oder ein Spektrometer mit Photonendetektor, typischerweise ein Polychromator und eine CCD-Kamera, sein.

Das Signal wird in Abhängigkeit von der Position des Scanners 4 und gegebenenfalls unter Berücksichtigung des Signals des Detektors 7 mit geeigneter Bildverarbeitung im Personalcomputer 17 flächenhaft und räumlich anschaulich aufbereitet.

Stellt die Optik 18 eine geeignete Abbildungsoptik dar, können auch 5 CCD-Kameras als Detektoren fungieren.

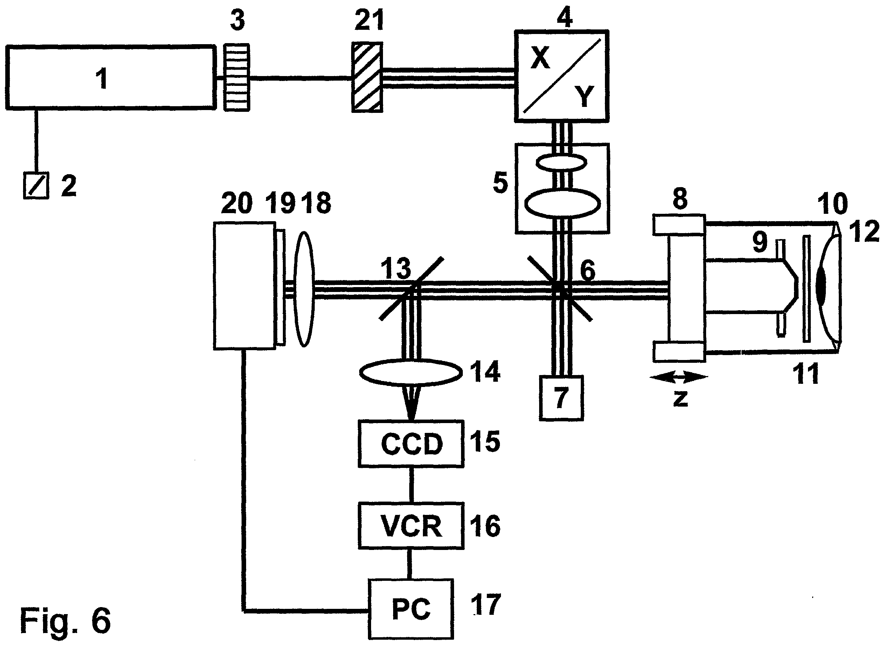

Zudem kann ein Modul 21, wie in Fig. 6 dargestellt, integriert sein, welcher anstelle des Abtastverfahrens mit nur einem Strahl auch das gleichzeitige oder nahezu gleichzeitige Abrastern mit mehreren Strahlen ermöglicht. Solch ein Modul 21 kann typischerweise in den Strahlengang des Lasers zwischen Schalter 3 und Scanner 4 integriert werden. Dieser Modul kann bekannte Multi-Linsen-Anordnungen oder Strahlenteiler beinhalten. Ein zeitlicher Versatz der Teilstrahlen im Femto- und Pikosekundenbereich ist ebenfalls möglich. Die Verteilung der Teilstrahlen im Target kann dabei günstigerweise eine Matrix in Form einer Rechteckfläche oder einer Kreisfläche oder in Form einer Linie sein. Im Modul 21 oder diesem im Strahlengang vor- oder nachgeschaltet kann ein vorzugsweise als Abschwächer wirksamer Leistungsregler angeordnet sein, um die durchgehende Laserstrahlung erfindungsgemäß vom "Bearbeitungsniveau" auf das "Diagnoseniveau" abzusenken.

### Bezugszeichenliste

- 1: Laser
- 2: Fußschalter
- 3: Schalter
- 4: x-y-Ablenksystem
- 5: Aufweitungsoptik
- 6: erster Strahlenteiler
- 7: Detektor zur Leistungsmessung und -Steuerung
- 8: z-Richtungs-Feinverstellung
- 9: Fokussierungsoptik
- 10: Glasfenster
- 11: mechanische Halterung
- 12: Auge
- 13: zweiter Strahlenteiler
- 14: Abbildungsoptik
- 15: Strahlungsdetektor für Remissionsstrahlung
- 16: Videorecorder
- 17: Personalcomputer
- 18: Optik
- 19: Filter
- 20: Strahlungsdetektor für Sekundärstrahlung
- 21: Modul zur Aufteilung und ggf. zeitl. Versetzung des Laserstrahles

## Patentansprüche

1. Vorrichtung zur minimal- bis nicht-invasiven optischen Bearbeitung und Erkennung von Geweben des Auges, mit einem gepulsten Laser (1) und einer Einrichtung zur Fokussierung der Laserstrahlung in einem zeilen-, flächenhaften oder räumlichen Muster, wobei der Bearbeitungsstrahlengang vom Laser (1) über einen schnellen Schalter (3), ein x-y-Ablenksystem (4), eine Aufweitungsoptik (5), einen ersten Strahlenteiler (6) und eine Fokussierungsoptik (9) mit einer z-Richtungs-Feinverstellung (8) zum Auge (12) des Patienten verläuft, wobei der erste Strahlenteiler (6) für einen Bruchteil der zum Auge (12) geleiteten Strahlung in Richtung eines Detektors (7) zur Leistungsmessung und -steuerung sowie für die vom Auge (12) kommende Strahlung in Richtung eines Auswertungsstrahlenganges durchlässig ist, wobei
der Schalter (3) eingerichtet ist, um die Leistung der Laserstrahlungspulse zwischen einem Bearbeitungspegel, bei dem eine therapeutische Wirkung erzielt wird, und einem Diagnosepegel, der geringer ist als der Bearbeitungspegel und bei dem intraokular im Korneagewebe durch Multi-Photonen-Anregung erzeugte Fluoreszenz entsteht, umzuschalten, wobei
- die Laserstrahlungspulse
- Folgefrequenzen im Megahertzbereich, und
- Pulsenergien im Pikojoulebereich oder im Nanojoulebereich haben, und
- auf einen Durchmesser kleiner als 5 µm fokussiert sind,
und die Vorrichtung weiterhin umfasst:
- Einrichtungen (6, 13, 15, 16, 17) zum Messen der genannten, durch Multi-Photonen-Anregung erzeugten Fluoreszenz.

2. Vorrichtung nach Anspruch 1, wobei Einrichtungen (8, 9) vorgesehen sind, um eine Fokusebene der Laserstrahlungspulse im Korneagewebe mit Nanometergenauigkeit zu variieren, um die optische Diagnose in unterschiedlichen Gewebetiefen durchzuführen, insbesondere in der Epithelschicht, der Bowman-Membran, und der Sklera.

3. Vorrichtung nach einem der Ansprüche 1 oder 2, wobei mit den Laserstrahlungspulsen im Diagnosepegel eine Zweiphotonen-Anregung der Fluoreszenz erfolgt.

4. Vorrichtung nach Anspruch 1, **dadurch gekennzeichnet, dass** der Schalter (3) zugleich ein Leistungsregler ist.

5. Vorrichtung nach einem der vorherigen Ansprüche, **gekennzeichnet durch** zwei Auswertestrahlungswege, die über einen Strahlteiler (13) eingeleitet sind, nämlich
- einen ersten Auswertestrahlungsweg zu einer Kamera (15) und
- einen zweiten Auswertestrahlungsweg zu einem Strahlungsdetektor (20).

6. Vorrichtung nach einem der vorherigen Ansprüche, **dadurch gekennzeichnet, dass** der Diagnosepegel unterhalb der Schwelle für Photodisruption im Korneagewebe liegt.

7. Vorrichtung nach Anspruch 1, wobei die Einrichtungen (6, 13, 15, 16, 17) zum Messen der genannten, durch Multi-Photonen-Anregung erzeugten Fluoreszenz umfassen:
einen Strahlenteiler (13) im Auswertungsstrahlengang zur Aufteilung der Remissions- und der Sekundärstrahlung auf jeweils dafür spezifische Strahlungsdetektoren (15 bzw. 20).

8. Vorrichtung nach Anspruch 7, wobei die Einrichtungen (6, 13, 15, 16, 17) zum Messen der genannten, durch Multi-Photonen-Anregung erzeugten Fluoreszenz eingerichtet sind, um die Ausgänge der Strahlungsdetektoren (15 bzw. 20) an gemeinsame Auswertungs- (17) und Anzeigeeinrichtungen (16) anzuschließen.

9. Vorrichtung nach Anspruch 8, wobei die Einrichtungen (6, 13, 15, 16, 17) zum Messen der genannten, durch Multi-Photonen-Anregung erzeugten Fluoreszenz eingerichtet sind, um auch den Ausgang des Detektors (7) zur Leistungsmessung und - steuerung an die gemeinsamen Auswertungs- (17) und Anzeigeeinrichtungen (16) anzuschließen.

10. Vorrichtung nach einem der Ansprüche 7 bis 9, wobei in den Einrichtungen (6, 13, 15, 16, 17) zum Messen der genannten, durch Multi-Photonen-Anregung erzeugten Fluoreszenz der Strahlungsdetektor (20) für die Sekundärstrahlung ein Photomultiplier ist.

11. Vorrichtung nach einem der Ansprüche 7 bis 9 wobei in den Einrichtungen (6, 13, 15, 16, 17) zum Messen der genannten, durch Multi-Photonen-Anregung erzeugten Fluoreszenz der Strahlungsdetektor (20) für die Sekundärstrahlung ein schneller Photomultiplier in Verbindung mit einem Einzelphotonenzähler mit einer Zeitauflösung in der Größenordnung von Picosekunden ist.

12. Vorrichtung nach einem der Ansprüche 7 bis 9, wobei in den Einrichtungen (6, 13, 15, 16, 17) zum Messen der genannten, durch Multi-Photonen-Anregung erzeugten Fluoreszenz der Strahlungsdetektor (20) für die Sekundärstrahlung ein Spektrometer mit Photonendetektor ist.

13. Vorrichtung nach Anspruch 12, wobei der Strahlungsdetektor (20) für die Sekundärstrahlung ein Polychromator in Verbindung mit einer CCD-Kamera ist.

14. Vorrichtung nach einem der Ansprüche 7 bis 13, weiterhin umfassen einen zwischen dem Schalter (3) und dem x-y-Ablenksystem (4) angeordneten Modul (21) zur Aufteilung des Laserstrahls in mehrere räumlich versetzte Einzelstrahlen.

15. Vorrichtung nach Anspruch 14, **dadurch gekennzeichnet, dass** der Modul (21) geeignet ist, die Einzelstrahlen auch in der Größenordnung von Femto- bis zu Picosekunden zeitlich zu versetzen.

## Claims

1. Apparatus for minimally invasive to non-invasive optical treatment and identification of tissues in the eye, comprising a pulsed laser (1) and a device for focusing the laser radiation in a line-like, extensive or spatial pattern, wherein the treatment beam path extends from the laser (1) to the eye (12) of the patient via a fast switch (3), an xy-deflection system (4), a widening optical unit (5), a first beam splitter (6) and a focusing optical unit (9) with a fine z-direction adjustment (8), wherein the first beam splitter (6) is transmissive to a fraction of the radiation guided to the eye (12) in the direction of a detector (7) for measuring and controlling power, and to the radiation coming from the eye (12) in the direction of an evaluation beam path, wherein
the switch (3) is configured to switch the power of the laser radiation pulses between a treatment level at which a therapeutic effect is obtained and a diagnostic level which is lower than the treatment level and at which fluorescence generated by multi-photon excitation arises intraocularly in the corneal tissue, wherein
- the laser radiation pulses
- have repetition rates in the megahertz range and
- pulse energies in the picojoule range or in the nanojoule range, and
- are focussed to a diameter smaller than 5 µm,
and the apparatus furthermore comprises:
- devices (6, 13, 15, 16, 17) for measuring the aforementioned fluorescence generated by multi-photon excitation.

2. Apparatus according to Claim 1, wherein devices (8, 9) are provided for varying a focal plane of the laser radiation pulses in the corneal tissue with nanometre accuracy in order to carry out the optical diagnosis at different tissue depths, in particular in the epithelial layer, the Bowman's membrane and the sclera.

3. Apparatus according to either of Claims 1 and 2, wherein there is a two-photon excitation of the fluorescence with the laser radiation pulses at the diagnostic level.

4. Apparatus according to Claim 1, **characterized in that** the switch (3) simultaneously is a power controller.

5. Apparatus according to one of the preceding claims, **characterized by** two evaluation radiation paths which are introduced by way of a beam splitter (13), specifically
- a first evaluation radiation path to a camera (15) and
- a second evaluation radiation path to radiation detector (20).

6. Apparatus according to one of the preceding claims, **characterized in that** the diagnostic level lies below the threshold for photodisruption in the corneal tissue.

7. Apparatus according to Claim 1, wherein the devices (6, 13, 15, 16, 17) for measuring the aforementioned fluorescence generated by multi-photon excitation comprise:
a beam splitter (13) in the evaluation beam path for splitting the diffusely reflected radiation and secondary radiation onto specific radiation detectors (15 and 20) in each case.

8. Apparatus according to Claim 7, wherein the devices (6, 13, 15, 16, 17) for measuring the aforementioned fluorescence generated by multi-photon excitation are configured to connect the outputs of the radiation detectors (15 and 20) to common evaluation (17) and indication devices (16).

9. Apparatus according to Claim 8, wherein the devices (6, 13, 15, 16, 17) for measuring the aforementioned fluorescence generated by multi-photon excitation are also configured to connect the output of the detector (7) for measuring and controlling power to the common evaluation (17) and indication (16) devices.

10. Apparatus according to one of Claims 7 to 9, wherein, in the devices (6, 13, 15, 16, 17) for measuring the aforementioned fluorescence generated by multi-photon excitation, the radiation detector (20) for the secondary radiation is a photomultiplier.

11. Apparatus according to one of Claims 7 to 9, wherein, in the devices (6, 13, 15, 16, 17) for measuring the aforementioned fluorescence generated by multi-photon excitation, the radiation detector (20) for the secondary radiation is a fast photomultiplier in conjunction with a single-photon counter with a time resolution in the order of picoseconds.

12. Apparatus according to one of Claims 7 to 9, wherein, in the devices (6, 13, 15, 16, 17) for measuring the aforementioned fluorescence generated by multi-photon excitation, the radiation detector (20) for the secondary radiation is a spectrometer with photon detector.

13. Apparatus according to Claim 12, wherein the radiation detector (20) for the secondary radiation is a polychromator in conjunction with a CCD camera.

14. Apparatus according to one of Claims 7 to 13, furthermore comprising a module (21), arranged between the switch (3) and the xy-deflection system (4), for dividing the laser beam into a plurality of spatially offset individual beams.

15. Apparatus according to Claim 14, **characterized in that** the module (21) is suitable for offsetting the individual beams over time, even in the order of femtoseconds to picoseconds.

## Revendications

1. Dispositif destiné au traitement optique invasif minimal à non invasif et au diagnostic de tissus oculaires, avec un laser (1) pulsé et un mécanisme destiné à la focalisation du rayon laser dans un modèle linéaire, plat ou spatial, dans lequel la trajectoire du faisceau de traitement du laser (1) est acheminée par l'intermédiaire d'un commutateur (3) rapide, d'un système de déviation de l'axe des abscisses et de l'axe des ordonnées (4), d'une optique d'élargissement (5), d'un premier séparateur de faisceau (6) et d'une optique de focalisation (9), avec un réglage de précision (8) de la direction de l'axe des cotes vers l'oeil (12) du patient,
dans lequel le premier séparateur de faisceau (6) est perméable pour une fraction du rayonnement dirigé vers l'oeil (12) dans la direction d'un détecteur (7) en vue de la mesure de la puissance et de la commande de la puissance, ainsi que pour le rayonnement provenant de l'oeil (12) dans la direction d'une trajectoire du faisceau d'évaluation, dans lequel le commutateur (3) est conçu en vue de faire basculer la puissance des impulsions du rayonnement laser entre un niveau destiné au traitement, pour lequel un effet thérapeutique est recherché, et un niveau destiné au diagnostic, lequel est plus faible que le niveau destiné au traitement et pour lequel est générée une fluorescence produite de manière intraoculaire dans le tissu cornéen, par l'intermédiaire d'une stimulation par multiples photons, dans lequel
- les impulsions du rayonnement laser
- ont des fréquences de répétition dans la gamme des mégahertz ; et
- ont des énergies d'impulsions dans la gamme des pico-joules ou dans la gamme des nanojoules ; et
- sont focalisées sur un diamètre inférieur à 5 µm,
et le dispositif comprend en outre :
- des mécanismes (6, 13, 15, 16, 17) destinés à la mesure de la fluorescence mentionnée, produite par l'intermédiaire d'une stimulation par multiples photons.

2. Dispositif selon la revendication 1, dans lequel des mécanismes (8, 9) sont prévus pour faire varier un plan focal des impulsions du rayon laser dans le tissu cornéen avec une précision de l'ordre du nanomètre, afin de réaliser le diagnostic optique dans différentes profondeurs de tissu, en particulier dans la couche de l'épithélium, dans la membrane de Bowman et dans la sclérotique.

3. Dispositif selon l'une des revendications 1 ou 2, dans lequel une stimulation à deux photons de la fluorescence a lieu au moyen des impulsions du rayon laser dans le niveau destiné au diagnostic.

4. Dispositif selon la revendication 1, **caractérisé en ce que** le commutateur (3) est à la fois un régulateur de puissance.

5. Dispositif selon l'une des revendications précédentes, **caractérisé par** deux trajectoires de rayonnement d'évaluation qui sont mises en oeuvre par l'intermédiaire d'un séparateur de faisceau (13), à savoir :
- une première trajectoire de rayonnement d'évaluation jusqu'à une caméra (15) ; et
- une deuxième trajectoire de rayonnement d'évaluation jusqu'à un détecteur de rayonnement (20).

6. Dispositif selon l'une des revendications précédentes, **caractérisé en ce que** le niveau destiné au diagnostic est situé au-dessous du seuil pour la photodisruption dans le tissu cornéen.

7. Dispositif selon la revendication 1, dans lequel les mécanismes (6, 13, 15, 16, 17) destinés à la mesure de la fluorescence mentionnée, produite par l'intermédiaire d'une stimulation par multiples photons, comprennent un séparateur de faisceau (13) dans la trajectoire du faisceau d'évaluation en vue de la division du rayonnement de rémission et du rayonnement secondaire sur les détecteurs de rayonnement (15 respectivement 20) respectifs, prévus spécifiquement à cet effet.

8. Dispositif selon la revendication 7, dans lequel les mécanismes (6, 13, 15, 16, 17) destinés à la mesure de la fluorescence mentionnée, produite par l'intermédiaire d'une stimulation par multiples photons, sont conçus en vue de raccorder les sorties des détecteurs de rayonnement (15 respectivement 20) à des mécanismes d'évaluation (17) et d'affichage (16) communs.

9. Dispositif selon la revendication 8, dans lequel les mécanismes (6, 13, 15, 16, 17) destinés à la mesure de la fluorescence mentionnée, produite par l'intermédiaire d'une stimulation par multiples photons, sont conçus en vue de raccorder également la sortie du détecteur (7) destiné à la mesure de la puissance et à la commande de la puissance aux mécanismes d'évaluation (17) et d'affichage (16) communs.

10. Dispositif selon l'une des revendications 7 à 9, dans lequel dans les mécanismes (6, 13, 15, 16, 17) destinés à la mesure de la fluorescence mentionnée, produite par l'intermédiaire d'une stimulation par multiples photons, le détecteur de rayonnement (20) pour le rayonnement secondaire est un photomultiplicateur.

11. Dispositif selon l'une des revendications 7 à 9, dans lequel dans les mécanismes (6, 13, 15, 16, 17) destinés à la mesure de la fluorescence mentionnée, produite par l'intermédiaire d'une stimulation par multiples photons, le détecteur de rayonnement (20) pour le rayonnement secondaire est un photomultiplicateur rapide en liaison avec un compteur de photons individuels dont la résolution temporelle est de l'ordre de grandeur de la picoseconde.

12. Dispositif selon l'une des revendications 7 à 9, dans lequel dans les mécanismes (6, 13, 15, 16, 17) destinés à la mesure de la fluorescence mentionnée, produite par l'intermédiaire d'une stimulation par multiples photons, le détecteur de rayonnement (20) pour le rayonnement secondaire est un spectromètre avec un détecteur de photons.

13. Dispositif selon la revendication 12, dans lequel le détecteur de rayonnement (20) pour le rayonnement secondaire est un polychromateur en liaison avec une caméra CCD.

14. Dispositif selon l'une des revendications 7 à 13, lequel comprend en outre un module (21) disposé entre le commutateur (3) et le système de déviation de l'axe des abscisses et de l'axe des ordonnées (4), en vue de la division du rayon laser en plusieurs rayons individuels décalés dans l'espace.

15. Dispositif selon la revendication 14, **caractérisé en ce que** le module (21) est adapté pour décaler également les rayons individuels dans le temps dans un ordre de grandeur allant de la femtoseconde à la picoseconde.
